# EUROPÄISCHE PATENTSCHRIFT

(11) **EP 1 943 952 B1**
(45) Veröffentlichungstag und Bekanntmachung des Hinweises auf die Patenterteilung: **26.10.2011**
(21) Anmeldenummer: 08100184.4
(22) Anmeldetag: 08.01.2008
(51) Int. Cl.: A61B 7/00, A61B 7/04, H04R 17/02, H04R 1/46

(54) **Medizinischer Schallsensor**
Medical sound wave sensor
Capteur médical de sons

(30) Priorität: 12.01.2007 DE 102007001921
(43) Veröffentlichungstag der Anmeldung: 16.07.2008
(73) Patentinhaber: Enverdis GmbH, 07745 Jena (DE)
(72) Erfinder: Hübner, Thomas, 07749 Jena (DE); Alt, Michael, 36275 Kirchheim (DE)
(74) Vertreter: von Kreisler Selting Werner

(56) Entgegenhaltungen:
- WO-A-97/28742
- WO-A-99/47044
- WO-A1-2007/061619
- DE-A1- 3 804 616
- DE-A1- 4 338 466
- US-A1- 2006 169 529
- "Technical Publication TP- 245; Cantilever Mounted PZT 5A Bimorphs®" [Online] 21. März 2006 (2006-03-21), , XP002475802 Gefunden im Internet: URL:http://web.archive.org/web/20060321221 027/http://www.morganelectroceramics.com/p dfs/tp245.pdf> [gefunden am 2008-04-09] * das ganze Dokument *

## Beschreibung

Die Erfindung betrifft einen medizinischen Schallsensor zur Detektion von Herzschall und/ oder Lungengeräuschen.

Zur Diagnose von Herz- und/ oder Lungenerkrankungen ist es bekannt, den Patienten mit einem Stethoskop abzuhören. Moderne Stethoskope weisen einen Schallsensor zur Detektion von Herzschall und/ oder Lungengeräuschen auf. Derartige Schallsensoren weisen beispielsweise ein Luftschall-Mikrofon auf, durch das der Luftschall in ein elektronisches Signal umgewandelt wird. Ebenso kann das Stethoskop einen Körperschall- bzw. Schwingungsschallsensor aufweisen.

WO 99/47044 beschreibt einen Sensor zur Messung des Pulsdrucks in einer Arterie, wobei ein piezoelektrisches Element über ein Verbindungselement mit einer Edelstahlmembran gekoppelt ist. Das andere Ende des piezoelektrischen Elements ist fest in dem Gehäuse des Sensors gehalten. Über die Edelstahlmembran werden Schwingungen an das piezoelektrische Element übertragen. Der Sensor ist hierbei zur Erfassung von Schwingungen zwischen 0,1 Hz und etwa 250 Hz geeignet.

Der Artikel "Technical Publication TP- 245 Cantilever Mounted PZT 5A Bimorphs®" (XP-002475802) vom 21. März 2006 und recherchierbar unter der URL http://web.archive.org/web/20060321221.de beschreibt verschiedene Arten von Bimorphstäben, die teilweise einseitig aufgehängt sind.

US 2006/0169529 beschreibt eine medizinische Vorrichtung zur Erfassung von Körperschall sowie eine Vorrichtung zur Verarbeitung der erfassten Signale.

DE 43 38 466 beschreibt ein Verfahren und eine Vorrichtung zur automatischen Erfassung auffälliger Atemgeräusche, wobei die Atemgeräusche in Echtzeit erfasst, aufbereitet und ausgewertet werden. Aus dem erfassten Frequenzbereich werden nur die vorher bestimmten charakteristischen Frequenzen bei der Auswertung berücksichtigt, an deren Position im Frequenzspektrum des Atemgeräusches des Probanden ein auffälliges Atemgeräusch durch ein signifikantes Merkmal zu erkennen ist.

WO 97/28742 beschreibt ein Stethoskop, mit dem Umgebungsgeräusche minimiert werden können.

DE 38 04 616 beschreibt ein Stethoskop, mit dem verschiedene Biosignale u.a. ein EKG erfasst und ausgewertet werden können. Verschiedene Pulssignale können über das EKG getriggert dargestellt werden.

WO 2007/061619 A1, die nachveröffentlichten Stand der Technik darstellt, beschreibt einen Sensor zum Messen bioakustischer Signale, wobei ein stabförmig ausgebildetes Schwingungselement zur Umwandlung mechanischer Schwingungen einer Membran in elektrische Signale verwendet wird, das auf piezoelektrischer Basis beruht. Das Ende des stabförmigen Schwingungselements kragt hierbei frei aus, so dass dieses freie Ende frei schwingen kann. Das Schwingungselement ist als Unimorphstab ausgebildet.

Aufgabe der Erfindung ist es, die Empfindlichkeit der existierenden Schallsensoren zu verbessern.

Die Lösung der Aufgabe erfolgt durch einen medizinischen Schwingungs- bzw. Schallsensor gemäß Anspruch 1.

Der erfindungsgemäße Schallsensor weist ein Sensorgehäuse auf. Eine Öffnung des Sensorgehäuses, die sich vorzugsweise im Wesentlichen über eine gesamte Seite des Sensors erstreckt, ist mit einer elastischen Membran überspannt. Die Membran wird zur Detektion von Schwingungen, die durch Herzklappen- und Wandbewegungen ausgelöst werden, sowie Herzschall und/oder Lungengeräuschen auf die Hautoberfläche des Patienten aufgelegt. Ferner weist der erfindungsgemäße Schallsensor ein insbesondere piezoelektrisches Schwingungselement zur Umwandlung der mechanischen Schwingungen der Membran in elektrische Signale auf. Das piezoelektrische Schwingungselement ist über ein Halteelement mit der Membran mittelbar oder unmittelbar verbunden. Hierbei trägt das Halteelement das Schwingungselement derart, dass das Schwingungselement durch mechanische Schwingungen der Membran in eine Schwingung versetzt werden kann, die sodann auf Grund des piezoelektrischen Charakters des Schwingungselements in elektrische Signale umgewandelt wird.

Auf Grund des erfindungsgemäßen Vorsehens eines piezoelektrischen Schwingungselements ist es möglich, dieses derart über das Halteelement zu lagern bzw. zu halten, dass bereits sehr geringe Schwingungen wahrgenommen werden können. Hierdurch ist die Sensibilität des medizinischen Schallsensors erheblich verbessert, so dass beispielsweise beim Einsatz des Schallsensors in einem Stethoskop Herzschall und/ oder Lungengeräusche deutlich differenzierbarer wahrgenommen und ausgewertet werden können. Hierdurch ist eine exaktere Diagnose möglich.

Erfindungsgemäß ist das Schwingungselement stabförmig ausgebildet. Aus dem Stand der Technik sind Schwingungselemente bekannt, die als Unimorphstab ausgebildet sind. Ein derartiger Unimorphstab weist eine piezoelektrische Komponente auf, die als Stab oder Schicht auf einen Träger aufgebracht wurde und von vorzugsweise zwei leitfähigen Schichten umgeben ist. Ein Unimorphstab funktioniert nach dem Prinzip der Piezoelektrizität, bei der lineare elektromechanische Wechselwirkungen , zwischen dem mechanischen und elektrischen Zustand in den Kristallen auftreten. Eine mechanische Verformung des Unimorphstabes führt hierbei zu einer Stauchung oder Dehnung der piezoelektrischen Komponente und damit zu einer proportionalen Änderung der elektrischen Polarisation durch die Trennung positiver und negativer Ladungen an der gegenüberliegenden Seite des Kristalls. Das hierbei entstehende Potential kann in Form einer Piezospannung im Kristall gemessen werden.

Erfindungsgemäß ist das Schwingungselement als Multimorphstab ausgebildet.

Zur Erzeugung einer möglichst hohen elektrischen Spannung eines Multimorphstabes ist die Richtung der Polarität der piezoelektrischen Komponenten geeignet zu wählen. Die piezoelektrische Polarität aller piezoelektrischen Komponenten, die bei einer bestimmten Auslenkung des Stabes gestaucht werden, ist gleichgerichtet, aber entgegengerichtet zu den piezoelektrischen Komponenten, die während dieser Auslenkung gedehnt werden.

Ein Trimorphstab als eine vorzugsweise Ausführungsform eines Schwingungselementes besteht aus einem z.B. leitfähigen Trägermaterial mit an zwei Seiten aufgebrachten piezoelektrischen Komponenten. Die Polarisierung wird zur Erzeugung einer möglichst hohen Signalspannung entgegengesetzt ausgerichtet. Die Signalspannung, die doppelt so hoch wie die erzeugte Spannung einer einzelnen piezoelektrischen Komponente ist, wird an den beiden Außenelektroden des Trimorphstabes abgegriffen.

Das erfindungsgemäß stabförmig ausgebildete Schwingungselement wird derart von dem Halteelement getragen, dass ein Ende frei auskragt und somit frei schwingen kann. Ggf. ist das Halteelement derart mit dem Schwingungselement verbunden, dass zwei freie Enden in Schwingungen versetzt werden können. Bevorzugt ist es, das insbesondere stabförmige Schwingungselement an einer Seite mit dem Halteelement zu verbinden, so dass das Schwingungselement einseitig aufgehängt ist. Zur Erhöhung der Signalspannung kann das Ende des Schwingungselementes durch ein kleines Gewicht beschwert werden.

Da die mechanische Verformung des Multimorphstabes in der Nähe seiner Aufhängung am Halteelement am größten ist, entsteht die höchste Signalspannung, wenn die leitfähige Beschichtung der piezoelektrischen Komponenten (Elektroden) nur in diesem Bereich aufgebracht wurde.

Bei einer besonders bevorzugten Ausführungsform ist als Schwingungselement ein Trimorphstab vorgesehen. Dieser weist zwei piezoelektrische Komponenten bzw. Bauteile auf, die beispielsweise quaderförmig ausgebildet sind. Zwischen den beiden piezoelektrischen Komponenten ist eine innere Schicht vorgesehen. Diese ist aus piezoelektrisch inaktivem Material bzw. Materialgemisch hergestellt. Erfindungswesentlich bei dem Vorsehen eines Trimorphstabes ist es, dass die innere Schicht elektrisch leitfähiges Material aufweist, insbesondere aus elektrisch leitfähigem Material besteht und/ oder an den Grenzflächen der inneren Schicht und den piezoelektrischen Komponenten eine elektrisch leitfähige Oberfläche vorgesehen ist. Die beiden elektrisch leitfähigen Oberflächen sind elektrisch miteinander verbunden. Vorzugsweise sind die beiden elektrisch leitfähigen Schichten im Bereich des Halteelements vorgesehen. Dies ist insbesondere vorteilhaft, da in diesem Bereich die größten mechanischen Verformungen des Trimorphstabes auftreten und somit entsprechend hohe Spannungssignale erzielt werden können.

In bevorzugter Ausführungsform ist das Schwingungselement einseitig aufgehängt bzw. als Kragarm ausgebildet und an einem Ende über das Halteelement schwingend gehalten. Ebenso ist es möglich, das Schwingungselement über ein einzelnes Halteelement derart zu halten, dass die beiden stabförmigen Enden des Schwingungselements über beide Seiten des Halteelements vorstehen. Insbesondere erfolgt eine mittige, wippenartige Lagerung des stabförmigen Schwingungselements über ein einziges Halteelement.

In einer bevorzugten Ausführungsform ist zwischen dem Halteelement und der Membran eine Kontaktplatte vorgesehen. Diese kann unmittelbar auf die Membran aufgeklebt sein. Durch das Vorsehen des Halteelements auf der Kontaktplatte, bei der es sich insbesondere um eine elektrische Leitungen aufweisende Kontaktplatte handelt, ist es möglich, das Schwingungselement auf einfache Weise an die entsprechenden Leitungen zur Übertragung der elektrischen Signale anzuschließen.

Bei dem Material der Membran handelt es sich vorzugsweise um elastisches, gummiartiges Material. Insbesondere weist die Membran biologische Werkstoffe, wie neopren-ähnliches Material auf. Um sicherzustellen, dass die Dämpfung der Membran möglichst gering ist, ist es bevorzugt, eine Membran zu verwenden, deren Dicke 0,5 mm oder weniger beträgt.

Um unkontrollierte Auslenkungen des Schwingungselements zu vermeiden, ist das Schwingungselement vorzugsweise innerhalb eines Dämpfungsmediums, wie eines Dämpfungsöls, angeordnet. Vorzugsweise sind die Dämpfungseigenschaften des Öls exakt gewählt und bekannt. Beispielsweise kann als Dämpfungsmedium ein Silikonöl, insbesondere ein Silikonölgemisch aus beispielsweise zwei Silikonölen, verwendet werden. Besonders geeignet ist ein Silikonölgemisch aus zwei Silikonölen im Verhältnis 2:1 mit einer Viskosität von 100 bzw. 1000 mm²/s. Vorzugsweise befindet sich das Dämpfungsmedium lediglich unterhalb des Unimorph- bzw. Multimorphstabes zwischen der Kontaktplatte und dem Stab. Auf Grund seiner Viskosität und der vorhandenen Kapillarkräfte fließt es nicht in den gesamten Raum des Innengehäuses.

Vorzugsweise ist innerhalb des Sensorgehäuses ein Innengehäuse angeordnet, das das Schwingungselement umschließt. Vorzugsweise umschließt das Innengehäuse das Schwingungselement dicht, so dass beim Vorsehen von flüssigem Dämpfungsmedium dieses innerhalb des Innengehäuses angeordnet ist. Vorzugsweise ist eine Seitenwand des Innengehäuses zumindest teilweise, vorzugsweise vollständig durch die Kontaktplatte ausgebildet. Um eine möglichst präzise Aufnahme von Herzschall und/ oder Lungengeräuschen zu ermöglichen, ist das Innengehäuse vorzugsweise ausschließlich mit der Membran verbunden. Ansonsten kann das Innengehäuse frei in dem Sensorgehäuse bewegt werden.

Bei einer bevorzugten Weiterbildung der Erfindung weist der medizinische Schallsensor mindestens ein Kontaktelement zum Detektieren des Aufsetzens des Schallsensors auf die Haut des Patienten auf. Durch Vorsehen eines derartigen Kontaktsensors, der vorzugsweise zwei entsprechend verschaltete Kontaktelemente aufweist, ist es beispielsweise möglich, ein automatisches Einschalten des Sensors zu realisieren. Beim Aufsetzen des Schallsensors treten üblicherweise laute Geräusche auf, die für einen Arzt äußerst unangenehm sind, wenn der Schallsensor in einem Stethoskop angeordnet ist. Es ist somit möglich, das Kontaktelement mit einer Steuerungseinrichtung zu verbinden und hierdurch eine Zeitverzögerung vorzusehen, so dass durch das Schwingungselement erzeugte Signale erst nach dem Aufsetzen des Sensors auf die Hautoberfläche abgegeben werden. Hierdurch ist das Auftreten von lauten Aufsetzgeräuschen vermieden. Um das Auftreten von Aufsetzgeräuschen oder anderen Artefaktgeräuschen zu vermeiden, kann ferner auch eine Stummschaltung in den erfindungsgemäßen Schallsensor bzw. eine mit dem Schallsensor verbundene Steuerungseinrichtung vorgesehen sein. Durch die Stummschaltung können somit Störgeräusche unterdrückt werden.

Bei einer weiteren bevorzugten Ausführungsform ist der medizinische Schallsensor mit mindestens einem, vorzugsweise drei Frequenzfiltern verbunden. Durch den bzw. die Frequenzfilter können Frequenzbereiche ausgefiltert werden, so dass nur noch ein Teil der von dem Schwingungselement aufgenommenen Schwingungen, die einem vordefinierten Frequenzbereich entsprechen, an einen mit dem Schallsensor verbundenen Ohrhörer weitergegeben werden. Besonders bevorzugt ist es hierbei, dass zwischen unterschiedlichen Frequenzbereichen gewählt werden kann. Hierzu weist der Schallsensor vorzugsweise einen Frequenzwahlschalter auf, der insbesondere mit dem Gehäuse des Schallsensors verbunden ist. Mit Hilfe des Frequenzwahlschalters kann somit beispielsweise zur Detektion von Herzschall eine Frequenz von 15 Hz - 600 Hz ausgewählt werden. Sollen mit dem Schallsensor Lungengeräusche detektiert werden, kann beispielsweise ein Frequenzbereich von 200 Hz - 5 KHz ausgewählt werden. In einem beispielsweise dritten auszuwählenden Bereich kann der gesamte Frequenzbereich ungefiltert detektiert werden, so dass ein Frequenzbereich von 15 Hz - 5 KHz detektiert wird.

Ferner kann der medizinische Schallsensor eine Lautstärkeregelung aufweisen. Dies ist insbesondere bei einem Vorsehen des Schallsensors in einem Stethoskop vorteilhaft.

Bei Vorsehen einer Batterie ist vorzugsweise eine Ladezustandsanzeige, beispielsweise in Form von LEDs, vorgesehen. Auch kann eine entsprechende Betriebszustandsanzeige (an/ aus) vorgesehen sein.

Bei einer weiteren besonders bevorzugten Ausführungsform sind mindestens zwei, vorzugsweise drei mit dem Sensorgehäuse verbundene Elektroden vorgesehen. Durch das Vorsehen von Elektroden kann zusätzlich zur Detektion von Herzschall und/ oder Lungengeräuschen ein EKG, insbesondere ein Ein-Kanal-EKG, aufgenommen werden. Bei Vorsehen von drei Elektroden wird vorzugsweise eine Elektrode zur Störunterdrückung verwendet. Hierdurch kann eine qualitativ sehr gute und nahezu störungsfreie Aufzeichnung der EKG-Signale erfolgen. Vorzugsweise sind die mindestens zwei Elektroden in einer Aufnahmeebene, bei der es sich insbesondere um die Membranebene handelt, angeordnet. Hierdurch ist es möglich, den Schallsensor gleichzeitig mit den Elektroden auf der Haut des Patienten aufzulegen und den Herzschall und/ oder Lungengeräusche gleichzeitig zum EKG aufzunehmen. Hierdurch ist es möglich, das EKG in Relation zu dem aufgenommenen Herzschall und/ oder Lungengeräuschen zu setzen. Insbesondere kann mit Hilfe des EKGs die R-Zacke genau bestimmt werden. Hierdurch kann eine genaue zeitliche Bestimmung jedes Herzschlags bestimmt und in zeitlichem Zusammenhang zu dem resultierenden Herzschall gesetzt werden.

Die Elektroden sowie die Kontaktelemente sind vorzugsweise auf einer Kreislinie angeordnet. Die Kreislinie umgibt vorzugsweise den Schallsensor, insbesondere die den Schall übertragende Membran.

Nachfolgend wird die Erfindung anhand einer bevorzugten Ausführungsform unter Bezugnahme auf die anliegenden Zeichnungen näher erläutert.

Es zeigen:
- Fig. 1: eine schematische perspektivische Ansicht eines Stethoskops,
- Fig. 2: eine schematische Ansicht des medizinischen Schallsensors von unten,
- Fig. 3: eine schematische Vorderansicht des medizinischen Schallsensors,
- Fig. 4: eine schematische Rückansicht des medizinischen Schallsensors,
- Fig. 5: eine schematische Schnittansicht des medizinischen Schallsensors, und
- Fig. 6: eine schematische Schnittansicht eines Trimorphstabes.

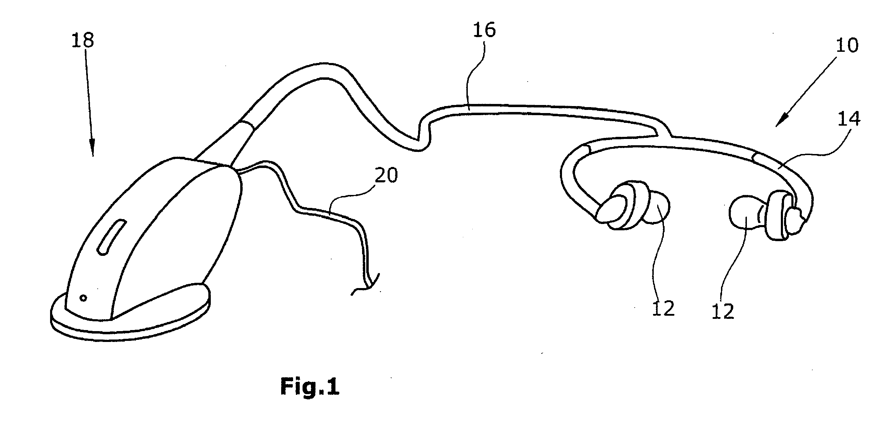

Ein Stethoskop **(****Fig. 1****)** weist einen Ohrhörer 10 mit Ohroliven 12 auf. In den Ohroliven 12 sind Lautsprecher angeordnet. Ferner weist der Ohrhörer 10 einen Bügel 14 auf, der die Ohroliven 12 trägt. Über ein Kabel 16 ist der Ohrhörer 10 mit einem Schallsensor 18 verbunden. Durch den Schallsensor oder weitere mit dem Schallsensor 18 verbundene Sensoren, wie Elektroden für ein EKG, erzeugte Signale können über das Kabel 16 zu den in den Ohroliven 12 angeordneten Lautsprechern geleitet werden. Ferner können die Signale über ein ebenfalls mit dem Sensor 18 verbundenes Kabel 20 zu einer Auswerteeinrichtung, wie einem Computer, gleitet werden. Die Signalübertragung erfolgt hierbei vorzugsweise digital. Ferner kann die Datenübertragung zu einer Auswerte- oder Steuereinheit drahtlos erfolgen.

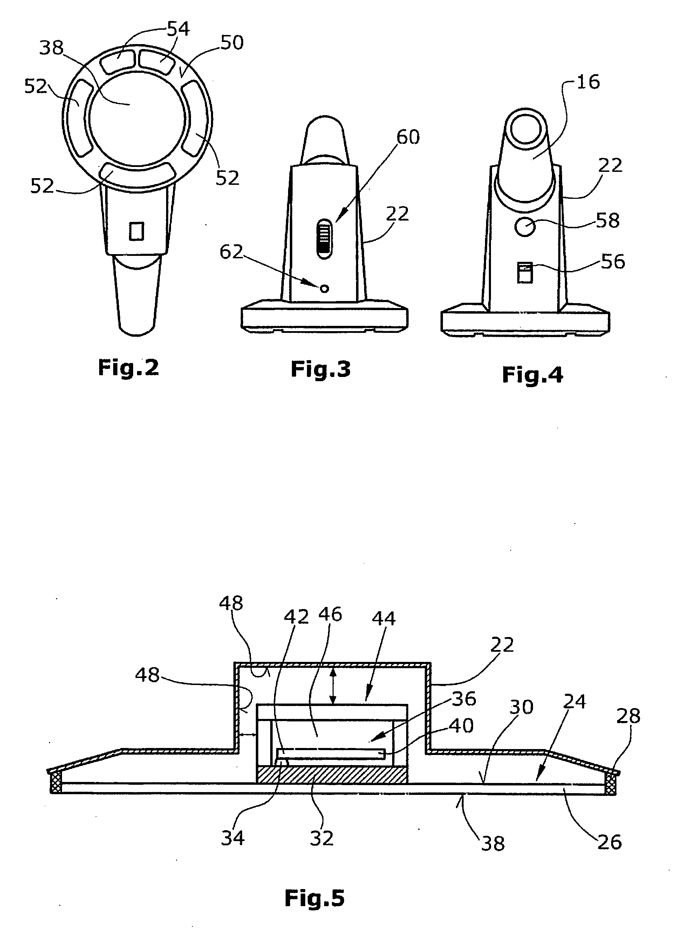

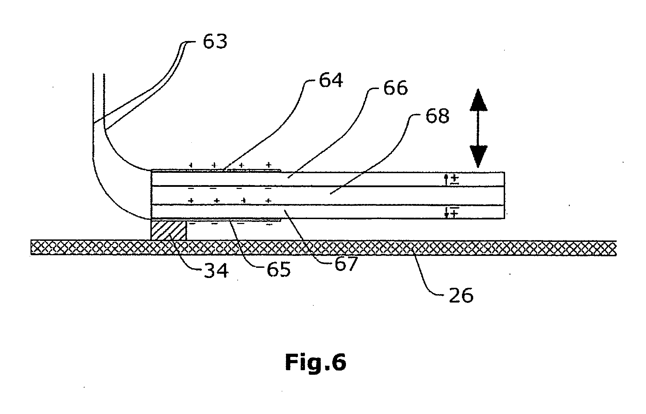

Der erfindungsgemäße medizinische Schallsensor 18 weist ein Sensorgehäuse 22 auf. Eine Öffnung 24 des Sensorgehäuses 22, die vorzugsweise kreisförmig ist und sich über die gesamte Seite des Sensorgehäuses 22 erstreckt, ist mit einer Membran 26 überspannt. Die Membran 26 ist somit fest mit einem ringförmigen Randelement 28 des Gehäuses 22 verbunden. Auf einer Innenseite 30 der Membran 26 ist eine Kontaktplatte 32, die elektrische Leiterbahnen aufweist, angeordnet. Die Kontaktplatte 32 ist beispielsweise auf die Innenseite 30 der Membran aufgeklebt. Mit der Oberseite der Kontaktplatte ist ein Halteelement 34 verbunden. Das Halteelement 34 trägt einen Uni- bzw. Multimorphstab, der als Schwingungselement 36 dient. Der Uni- bzw. Multimorphstab 36 ist über elektrische Leitungen 63 **(****Fig.6****)** mit der Kontaktplatte verbunden. Die Kontaktplatte ist über in **Fig. 5** nicht dargestellte elektrische Leitungen mit dem Kabel 20 **(****Fig. 1****)** verbunden.

In **Fig.6** ist beispielhaft der Aufbau eines als Trimorphstab ausgebildeten Schwingungselementes 36 dargestellt. Der Trimorphstab ist im abgebildeten Beispiel über das Halteelement 34 direkt mit der Membran 26 verbunden. Er besteht aus einem piezoelektrisch inaktiven Trägermaterial 68, das im vorliegenden Fall elektrisch leitend ist. Mit diesem Trägermaterial 68 sind zwei piezoelektrisch aktive Komponenten 66 und 67 verbunden, die piezoelektrisch entgegengesetzt polarisiert sind. Da die piezoelektrische Komponenten 66 bei Biegung (durch dicken Doppelpfeil symbolisch dargestellt) des Stabes dann gedehnt wird, wenn die Komponente 67 gestaucht wird, addieren sich - wie durch die Polariät der leitfähigen Schichten 64 und 65 dargestellt - die Piezospannungen. Die addierte Spannung kann mittels der elektrischen Verbindungen 63 abgegriffen werden. In **Fig.6** wurden die elektrisch leitfähigen Schichten 64 und 65 lediglich im Bereich des Halteelementes 34 aufgebracht, da in diesem Bereich die mechanische Belastung der piezoelektrischen Komponenten am größten ist und damit eine besonders hohe elektrische Signalspannung über die elektrischen Verbindungen 63 abgegriffen werden kann. Falls das Trägermaterial 68 des Trimorphstabes aus einem nicht elektrisch leitenden Material besteht, sind die an den inneren Flächen der piezoelektrischen Komponenten 66 und 67 erzeugten Ladungen durch elektrisch leitfähige Schichten miteinander zu verbinden.

Sobald die äußere Seite 38 der Membran 26 auf eine Patientenoberfläche aufgelegt wird, wird die Membran 26 durch den Herzschall oder Lungengeräusche in Schwingungen versetzt. Diese werden auf das Schwingungselement 36 übertragen, wobei im dargestellten Ausführungsbeispiel insbesondere ein freies Ende 40 des Schwingungselements 36 frei schwingen kann. Hierzu ist im dargestellten Ausführungsbeispiel das gegenüberliegende Ende 42 des vorzugsweise stabförmigen Schwingungselements mit dem Halteelement 34 verbunden.

Das Schwingungselement 36 ist von einem Innengehäuse 44 umgeben, das im dargestellten Ausführungsbeispiel quaderförmig ausgebildet ist. Das Innengehäuse 44 umgibt das Schwingungselement 36 vollständig, wobei eine Seitenfläche des quaderförmigen Innengehäuses 44 von der Kontaktplatte 32 gebildet ist. Das Innengehäuse weist somit einen dicht abgeschlossenen Hohlraum 46 auf. Es ist möglich, den Hohlraum 46 mit einem Dämpfungsmedium, insbesondere einem Dämpfungsöl, zu füllen, so dass das Dämpfungsöl das Schwingungselement 36 vollständig umgibt. Im Allgemeinen genügt es aber auch, wenn sich ein Tropfen des Silikonöls im Bereich des freien Endes 40 des Schwingungselements 36 befindet.

Das Innengehäuse 44 ist ausschließlich mit der Membran 26 verbunden und weist im Übrigen zu den Innenseiten 48 des Sensorgehäuses 22 einen Abstand auf, so dass sich das Innengehäuse 44 entsprechend der auf die Membran 26 wirkenden mechanischen Schwingungen frei bewegen kann.

In **Fig. 2 - 4** sind weitere Elemente des erfindungsgemäßen Sensors entnehmbar, die zur Verdeutlichung der Erfindung in **Fig. 5** nicht dargestellt sind.

An einer Unterseite 50 des Sensors, die in derselben Ebene wie die Unterseite 38 der Membran 26 liegt, sind drei Elektroden 52 versetzt um die Membran 26 herum angeordnet. Die Elektroden 52 sind mit elektrischen Leitungen verbunden, die beispielsweise über das Kabel 20 **(****Fig. 1****)** mit einem Computer verbunden sind. Ebenso können die Elektroden 52 mit einer Steuereinrichtung, die insbesondere auch innerhalb des Gehäuses 22 angeordnet sein kann, verbunden sein. An der die Membran 26 ringförmig umgebenden Unterseite 50 ist ferner ein Kontaktelement 54 vorgesehen. Durch das Kontaktelement 54 wird das Aufsetzen des Sensors auf die Hautoberfläche des Patienten detektiert.

Innerhalb des Gehäuses 22 ist eine Steuereinrichtung, die insbesondere einen Frequenzfilter aufweist, vorgesehen. Selbstverständlich kann die Steuereinrichtung auch außerhalb des Sensorgehäuses 22 angeordnet sein. Ferner kann der innerhalb des Gehäuses 22 vorgesehene Frequenzfilter mit einem Frequenzwahlschalter 56, der an einer Rückseite des Gehäuses 22 (**Fig. 4**) vorgesehen ist, verbunden sein. Mit dem Frequenzwahlschalter 56 können unterschiedliche Frequenzen, die beispielsweise auf den Ohrhörer 10 übertragen werden, ausgewählt werden.

Ferner kann an der Rückseite (**Fig. 4**) ein Stecker 58 vorgesehen sein, in den das Kabel 20 **(****Fig. 1****),** das mit einer Auswerteeinrichtung, wie einem PC, verbunden sein kann, einsteckbar ist.

An der Vorderseite **(****Fig. 3****)** des Sensorgehäuses 22 kann ein Lautstärkeregler 60 zur Regelung der Lautstärke des auf den Ohrhörer (10) übertragenen Schalls angeordnet sein. Ferner kann an der Vorderseite eine LED 62 zur Anzeige des Ladezustands und/ oder des Betriebszustands vorgesehen sein.

## Patentansprüche

1. Medizinischer Schallsensor zur Detektion von Herzschall und/ oder Lungengeräuschen bzw. Schwingungen, mit
einem Sensorgehäuse (22) mit einer Öffnung (24), die von einer elastischen Membran (26) überspannt ist,
einem stabförmig ausgebildeten Schwingungselement (36) zur Umwandlung mechanischer Schwingungen der Membran (26) in elektrische Signale, das auf piezoelektrischer Basis beruht,
einem mit der Membran (26) verbundenen Halteelement (34), das das Schwingungselement (36) trägt,
**dadurch gekennzeichnet, dass**
ein Ende (40) des stabförmigen Schwingungselements (36) derart frei auskragt, dass dieses freie Ende (40) frei schwingen kann, und
das stabförmig ausgebildete Schwingungselement (36) als Multimorphstab ausgebildet ist, wobei die piezoelektrische Polarität aller piezoelektrischen Komponenten, die bei einer bestimmten Auslenkung des Stabes gestaucht werden, gleichgerichtet sind, aber entgegengerichtet sind zur Polarität derjenigen piezoelektrischen Komponenten, die während dieser Auslenkung gedehnt werden.

2. Medizinischer Schallsensor nach Anspruch 1, **dadurch gekennzeichnet, dass** die piezoelektrischen Komponenten des Schwingungselements (36) jeweils an mindestens einer Seite eine leitfähigen Schicht (Elektrode) besitzen.

3. Medizinischer Schallsensor nach Anspruch 2 , **dadurch gekennzeichnet, dass** die leitfähige(n) Schicht(en) nur einen Teil der piezoelektrischen Plattenelemente im Bereich des Haltelements (34) überdecken.

4. Medizinischer Schallsensor nach einem der Ansprüche 1 - 3, **dadurch gekennzeichnet, dass** als Multimorphstab ein Trimorphstab vorgesehen ist, wobei eine zwischen zwei Komponenten (66, 67) angeordnete innere piezoelektrisch inaktive Schicht (68) entweder elektrisch leitfähiges Material aufweist und/ oder an der Grenzfläche zu den piezoelektrischen Komponenten (66, 67) zumindest im Bereich des Halteelements (34) eine elektrisch leitfähige Oberfläche aufweist und die beiden leitfähigen Oberflächen elektrisch miteinander verbunden sind.

5. Medizinischer Schallsensor nach einem der Ansprüche 1 - 4, **dadurch gekennzeichnet, dass** das insbesondere stabförmige Schwingungselement (36) derart mit dem Halteelement (34) verbunden ist, dass es einseitig aufgehängt ist.

6. Medizinischer Schallsensor nach einem der Ansprüche 1 - 5, **dadurch gekennzeichnet, dass** das stabförmige Schwingungselement (36) derart mit dem Halteelement (34) verbunden ist, dass es beidseitig auskragt.

7. Medizinischer Schallsensor nach einem der Ansprüche 1 - 6, **dadurch gekennzeichnet, dass** zwischen dem Halteelement (34) und der Membran (26) eine Kontaktfläche (32) vorgesehen ist.

8. Medizinischer Schallsensor nach Anspruch 7, **dadurch gekennzeichnet, dass** ein Dämpfungsmedium, insbesondere eines Dämpfungsöl, zwischen dem Schwingungselement (36) und der Kontaktfläche (32) angeordnet ist.

9. Medizinischer Schallsensor nach einem der Ansprüche 1 - 8, **dadurch gekennzeichnet, dass** das eine oder beide Enden des stabförmigen Schwingungselementes (36) durch ein Gewicht beschwert sind.

10. Medizinischer Schallsensor nach einem der Ansprüche 7 oder 8, **gekennzeichnet durch** ein innerhalb des Sensorgehäuses (22) angeordnetes Innengehäuse (44), welches das Schwingungselement (36) umschließt.

11. Medizinischer Schallsensor nach Anspruch 10, **dadurch gekennzeichnet, dass** eine Seitenwand des Innengehäuses (44) zumindest teilweise durch die Kontaktfläche (32) ausgebildet ist.

12. Medizinischer Schallsensor nach Anspruch 10 oder 11, **dadurch gekennzeichnet, dass** das Innengehäuse (44) ausschließlich mit der Membran (26) verbunden ist.

13. Medizinischer Schallsensor nach einem der Ansprüche 1 - 12, **gekennzeichnet durch** ein Kontaktelement (54) zum Detektieren des Aufsetzens des Schallsensors auf die Haut.

14. Medizinischer Schallsensor nach einem der Ansprüche 1 - 13, **gekennzeichnet durch** einen Frequenzfilter zum Ausfiltern eines Frequenzbereichs.

15. Medizinischer Schallsensor nach Anspruch 14, **dadurch gekennzeichnet, dass** der ausgefilterte Frequenzbereich des Frequenzfilters einstellbar ist, insbesondere über einen am Sensorgehäuse (22) angeordneten Frequenzwahlschalter (56).

16. Medizinischer Schallsensor nach einem der Ansprüche 1 - 15, **gekennzeichnet durch** ein Stummschaltelement zum Unterdrücken von Störgeräuschen.

17. Medizinischer Schallsensor nach einem der Ansprüche 1 - 16, **gekennzeichnet durch** mindestens zwei mit dem Sensorgehäuse (22) verbundene Elektroden (52) zur Aufnahme eines EKGs.

18. Medizinischer Schallsensor nach Anspruch 17, **dadurch gekennzeichnet, dass** die mindestens zwei Elektroden (52) in einer Auflageebene (50) der Membran (26) angeordnet sind.

## Claims

1. A medical sound wave sensor for detecting heart sound and/or lung sounds or vibrations, comprising
a sensor housing (22) having an opening (24) over which an elastic membrane (26) is stretched,
a rod-shaped vibration element (36) for transforming mechanical vibrations of the membrane (26) into electric signals, which operates on a piezoelectric basis,
a support element (34) connected to the membrane (26), which supports the vibration element (36),
**characterized in that**
an end of (40) of the rod-shaped vibration element (36) is cantilevered such that this free end (40) can vibrate freely, and
the rod-shaped vibration element (36) is configured as a multimorph rod, the piezoelectric polarity of all piezoelectric components that are compressed upon a certain deflection of the rod has the same orientation, but is opposite to the polarity of those piezoelectric components that are stretched during this deflection.

2. The medical sound wave sensor of claim 1, **characterized in that** the piezoelectric components of the vibration element (36) each have a conductive layer (electrode) on at least one side thereof.

3. The medical sound wave sensor of claim 2, **characterized in that** the conductive layer(s) cover(s) only a part of the piezoelectric plate elements in the region of the support element (34).

4. The medical sound wave sensor of one of claims 1 - 3, **characterized in that** a trimorph rod is provided as the multimorph rod, wherein an inner, piezoelectrically inactive layer (68) arranged between two components (66, 67) either comprises electrically conductive material and/or has an electrically conductive surface at the interface with the piezoelectric components (66, 67) at least in the region of the support element (34), and wherein the two conductive surfaces are electrically interconnected.

5. The medical sound wave sensor of one of claims 1-4, **characterized in that** the in particular rod-shaped vibration element (36) is connected to the support element (34) such that it is suspended on one side.

6. The medical sound wave sensor of one of claims 1-5, **characterized in that** the rod-shaped vibration element (36) is connected to the support element (34) such that it is cantilevered on both sides.

7. The medical sound wave sensor of one of claims 1-6, **characterized in that** a contact surface (32) is provided between the support element (34) and the membrane (26).

8. The medical sound wave sensor of claim 7, **characterized in that** a damping medium, in particular a damping oil, is provided between the vibration element (36) and the contact surface (32).

9. The medical sound wave sensor of one of claims 1-8, **characterized in that** one or both ends of the rod-shaped vibration element (36) are loaded with a weight.

10. The medical sound wave sensor of one of claims 7 or 8, **characterized by** an inner housing (44) arranged within the sensor housing (22), said inner housing enclosing the vibration element (36).

11. The medical sound wave sensor of claim 10, **characterized in that** a side wall of the inner housing (44) is at least partly formed by the contact surface (32).

12. The medical sound wave sensor of one of claims 10 or 11, **characterized in that** the inner housing (44) is exclusively connected to the membrane (26).

13. The medical sound wave sensor of one of claims 1-12, **characterized by** a contact element (54) for detecting the application of the sound wave sensor onto the skin.

14. The medical sound wave sensor of one of claims 1-13, **characterized by** a frequency filter for filtering out a frequency range.

15. The medical sound wave sensor of claim 14, **characterized in that** the filtered-out frequency range of the frequency filter is adjustable, in particular by means of a frequency selection switch (56) provided on the sensor housing (22).

16. The medical sound wave sensor of one of claims 1-15, **characterized by** a mute switch element for suppressing noises.

17. The medical sound wave sensor of one of claims 1-16, **characterized by** at least two electrodes (52) for recording an ECG, said electrodes being connected to the sensor housing (22).

18. The medical sound wave sensor of claim 17, **characterized in that** the at least two electrodes (52) are arranged in the plane of rest (50) of the membrane (26).

## Revendications

1. Capteur médical de sons pour la détection de sons de coeur et/ou de sons des poumons, ou de vibrations, comprenant
un boitier de capteur (22) avec une ouverture (24) à travers laquelle est tendue une membrane élastique (26),
un élément de vibration (36) en forme de bâton destiné à transformer les vibrations mécaniques de ladite membrane (26) en signaux électriques, l'élément se basant sur la piézoélectricité,
un élément de support (34) lié à ladite membrane (26) et supportant ledit élément de vibration (36),
**caractérisé en ce qu'**
une extrémité (40) dudit élément de vibration (36) en forme de bâton saille librement de sorte que cette extrémité libre (40) peut vibrer librement, et
ledit élément de vibration (36) en forme de bâton est un bâton polymorphe, la polarité piézoélectrique de tous les composants piézoélectriques qui sont comprimés lors d'une certaine déviation du bâton est orientée dans le même sens, tandis qu'elle est orientée dans le sens opposé à la polarité des composants piézoélectriques qui sont étendus lors de cette déviation.

2. Capteur médical de sons selon la revendication 1, **caractérisé en ce que** chaque composant piézoélectrique dudit élément de vibration (36) comprend une couche conductrice (électrode) sur au moins un côté.

3. Capteur médical de sons selon la revendication 2, **caractérisé en ce que** la (les) couche(s) conductrice(s) ne couvre(nt) qu'une partie des éléments de plaques piézoélectriques dans la région dudit élément de support (34).

4. Capteur médical de sons selon l'une des revendications 1-3, **caractérisé en ce qu'**un bâton trimorphe est prévu comme bâton polymorphe, où une couche interne piézoélectriquement inactive (68), disposée entre deux composants (66, 67), comprend un matériau électriquement conducteur et/ou comprend, au moins dans la région dudit élément de support (34) une surface électriquement conductrice située à la face de limite vers lesdits composants piézoélectriques (66, 67), et les deux surfaces conductrices sont liées électriquement entre elles.

5. Capteur médical de sons selon l'une des revendications 1-4, **caractérisé en ce que** ledit élément de vibration (36), notamment en forme de bâton, est lié audit élément de support (34) de sorte qu'il est suspendu à un côté.

6. Capteur médical de sons selon l'une des revendications 1-5, **caractérisé en ce que** ledit élément de vibration (36) en forme de bâton est lié audit élément de support (34) de sorte qu'il saille des deux côtés.

7. Capteur médical de sons selon l'une des revendications 1-6, **caractérisé en ce qu'**une face de contact (32) est prévue entre ledit élément de support (34) et ladite membrane (26).

8. Capteur médical de sons selon la revendication 7, **caractérisé en ce qu'**un médium amortisseur, notamment un huile amortisseur, est prévu entre ledit élément de vibration (36) et ladite face de contact (32).

9. Capteur médical de sons selon l'une des revendications 1-8, **caractérisé en ce que** l'une ou les deux extrémités dudit élément de vibration (36) en forme bâton sont chargées avec un poids.

10. Capteur médical de sons selon les revendications 7 ou 8, **caractérisé par** un boitier intérieur (44) disposé dans ledit boitier de capteur (22) et enfermant ledit élément de vibration (36).

11. Capteur médical de sons selon la revendication 10, **caractérisé en ce qu'**une paroi latérale dudit boitier intérieur (44) est au moins partiellement formée par ladite face de contact (32).

12. Capteur médical de sons selon les revendications 10 ou 11, **caractérisé en ce que** ledit boitier intérieur (44) est seulement lié à ladite membrane (26).

13. Capteur médical de sons selon l'une des revendications 1-12, **caractérisé par** un élément de contact (54) destiné à la détection de l'application duit capteur de sons sur la peau.

14. Capteur médical de sons selon l'une des revendications 1-13, **caractérisé par** un filtre de fréquence pour filtrer une gamme des fréquences.

15. Capteur médical de sons selon la revendication 14, **caractérisé en ce que** la gamme des fréquences filtrée est réglable, notamment à l'aide d'un sélecteur de fréquence (56) prévu sur ledit boitier de capteur (22).

16. Capteur médical de sons selon l'une des revendications 1-15, **caractérisé par** un élément silencieux destiné à supprimer des bruits.

17. Capteur médical de sons selon l'une des revendications 1-16, **caractérisé par** au moins deux électrodes (52) liées audit boitier de capteur (22), lesdites électrodes servant à enregistrer une ECG.

18. Capteur médical de sons selon la revendication 17, **caractérisé en ce que** lesdites au moins deux électrodes (52) sont disposées dans un plan de repos (50) de la membrane (26).
